# EUROPEAN PATENT SPECIFICATION

(11) **EP 1 620 403 B1**
(45) Date of publication and mention of the grant of the patent: **28.11.2007**
(21) Application number: 04729420.2
(22) Date of filing: 26.04.2004
(51) Int. Cl.: C07D 211/78, A61K 31/451

(54) **NOVEL 3,4-DISUBSTITUTED 1,2,3,6-TETRAHYDROPYRIDINE DERIVATIVES**
3,4-DISUBSTITUIERTE 1,2,3,4-TETRAHYDROPYRIDINDERIVATE
NOUVEAUX DERIVES DE LA 1,2,3,6-TETRAHYDROPYRIDINE 3,4-DISUBSTITUEE

(30) Priority: 29.04.2003 EP 03004446
(43) Date of publication of application: 01.02.2006
(73) Proprietor: Actelion Pharmaceuticals Ltd., 4123 Allschwil (CH)
(72) Inventor: BEZENCON, Olivier, CH-4125 Riehen (CH); BUR, Daniel, CH-4106 Therwil (CH); FISCHLI, Walter, CH-4123 Allschwil (CH); REMEN, Lubos, CH-4123 Allschwil (CH); RICHARD-BILDSTEIN, Sylvia, F-68440 Dietwiller (FR); WELLER, Thomas, CH-4102 Binningen (CH)
(74) Representative: Schager, Frank
(86) International application number: PCT/EP2004/004370
(87) International publication number: WO 2004/096769

(56) References cited:
- WO-A-20/04002957
- WO-A1-97/09311

## Description

The invention relates to novel compounds of the general formula **I**. The invention also concerns pharmaceutical compositions containing one or more compounds of formula **I**. The compounds of formula **I** are especially useful as renin inhibitors in cardiovascular events and renal insufficiency. Furthermore, some of these compounds can be regarded as inhibitors of other aspartyl proteases and might therefore be useful as inhibitors of plasmepsins to treat malaria and as inhibitors of *Candida albicans* secreted aspartyl proteases to treat fungal infections.

In the renin-angiotensin system (RAS) the biologically active angiotensin II (Ang II) is generated by a two-step mechanism. The highly specific enzyme renin cleaves angiotensinogen to angiotensin I (Ang I), which is then further processed to Ang II by the less specific angiotensin-converting enzyme (ACE). Ang II is known to work on at least two receptor subtypes called AT₁ and AT₂. Whereas AT₁ seems to transmit most of the known functions of Ang II, the role of AT₂ is still unknown.

Modulation of the RAS represents a major advance in the treatment of cardiovascular diseases. ACE inhibitors and AT₁ blockers have been accepted to treat hypertension (Waeber B. et al., "The renin-angiotensin system: role in experimental and human hypertension", in Berkenhager W. H., Reid J. L. (eds): Hypertension, Amsterdam, Elsevier Science Publishing Co, 1996, 489-519; Weber M. A., Am. J. Hypertens., 1992, 5, 247S). In addition, ACE inhibitors are used for renal protection (Rosenberg M. E. et al., Kidney International, 1994, 45, 403; Breyer J. A. et al., Kidney International, 1994, 45, S156), in the prevention of congestive heart failure (Vaughan D. E. et al., Cardiovasc. Res., 1994, 28, 159; Fouad-Tarazi F. et al., Am. J. Med., 1988, 84 (Suppl. 3A), 83) and myocardial infarction (Pfeffer M. A. et al., N. Engl. J. Med., 1992, 327, 669).

The rationale to develop renin inhibitors is the specificity of renin (Kleinert H. D., Cardiovasc. Drugs, 1995, 9, 645). The only substrate known for renin is angiotensinogen, which can only be processed (under physiological conditions) by renin. In contrast, ACE can also cleave bradykinin besides Ang I and can be bypassed by chymase, a serine protease (Husain A., J. Hypertens., 1993, 11, 1155). In patients inhibition of ACE thus leads to bradykinin accumulation causing cough (5-20%) and potentially life-threatening angioneurotic edema (0.1-0.2%) (Israili Z. H. et al., Annals of Internal Medicine, 1992, 117, 234). Chymase is not inhibited by ACE inhibitors. Therefore, the formation of Ang II is still possible in patients treated with ACE inhibitors. Blockade of the AT₁ receptor (e.g. by losartan) on the other hand overexposes other AT-receptor subtypes to Ang II, whose concentration is dramatically increased by the blockade of AT₁ receptors. This may raise serious questions regarding the safety and efficacy profile of AT₁ receptor antagonists. In summary, renin inhibitors are not only expected to be different from ACE inhibitors and AT₁ blockers with regard to safety, but more importantly also with regard to their efficacy to block the RAS.

Only limited clinical experience (Azizi M. et al., J. Hypertens., 1994, 12, 419; Neutel J. M. et al., Am. Heart, 1991, 122, 1094) has been created with renin inhibitors because of their insufficient oral activity due to their peptidomimetic character (Kleinert H. D., Cardiovasc. Drugs, 1995, 9, 645). The clinical development of several compounds has been stopped because of this problem together with the high cost of goods. Only one compound containing four chiral centers has entered clinical trials (Rahuel J. et al., Chem. Biol., 2000, 7, 493; Mealy N. E., Drugs of the Future, 2001, 26, 1139). Thus, metabolically stable, orally bioavailable and sufficiently soluble renin inhibitors that can be prepared on a large scale are missing and sought. Recently, the first non-peptide renin inhibitors were described which show high *in vitro* activity (Oefner C. et al., Chem. Biol., 1999, 6, 127; Patent Application WO97/09311; Märki H. P. et al., Il Farmaco, 2001, 56, 21). However, the development status of these compounds is not known. WO97/0931 describes 4-(oxyalkoxyphenyl)-3-oxy-piperidines acting as renin inhibitors.

The present invention relates to the unexpected identification of renin inhibitors of a non-peptidic nature and of low molecular weight. Orally active renin inhibitors of long duration of action which are active in indications beyond blood pressure regulation where the tissular renin-chymase system may be activated leading to pathophysiologically altered local functions such as renal, cardiac and vascular remodeling, atherosclerosis, and possibly restenosis, are described.

In particular, the present invention relates to novel compounds of the general formula **I** 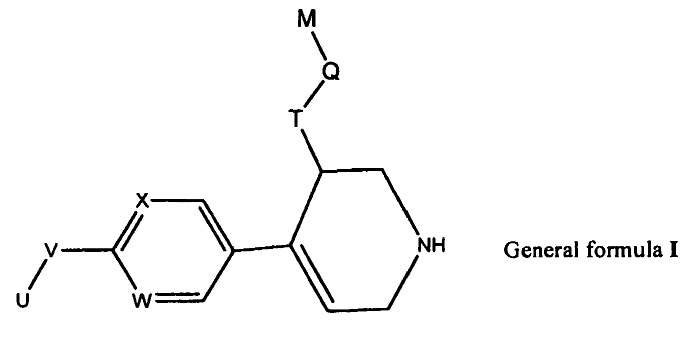 wherein
X and W represent independently a nitrogen atom or a CH-group;
V represents -(CH₂)ᵣ-; -A-(CH₂)ₛ-; -CH₂-A-(CH₂)ₜ-; -(CH₂)ₛ-A-; -(CH₂)₂-A-(CH₂)ᵤ-; -A-(CH₂)ᵥ-B-; -CH₂-CH₂-CH₂-A-CH₂-; -A-CH₂-CH₂-B-CH₂-; -CH₂-A-CH₂-CH₂-B-; -CH₂-CH₂-CH₂-A-CH₂-CH₂-; -CH₂-CH₂-CH₂-CH₂-A-CH₂-; -A-CH₂-CH₂-B-CH₂-CH₂-; -CH₂-A-CH₂-CH₂-B-CH₂-; -CH₂-A-CH₂-CH₂-CH₂-B-; or -CH₂-CH₂-A-CH₂-CH₂-B-;
A and B independently represent -O-; -S-; -SO-; or -SO₂-;
U represents aryl; or heteroaryl;
T represents -CONR¹-; -(CH₂)pOCO-; -(CH₂)ₚN(R¹)CO-; -(CH₂)ₚN(R¹)SO₂-; -COO-; -(CH₂)ₚOCONR¹-; or -(CH₂)ₚN(R¹')CONR¹-;
Q represents lower alkylene; or lower alkenylene;
M represents hydrogen; cycloalkyl; aryl; heterocyclyl; heteroaryl; aryl-O(CH₂)ᵥR²; heteroaryl-O(CH₂)ᵥR²; aryl-O(CH₂)₂O(CH₂)_{w}R²; heteroaryl-(CH₂)₂O(CH₂)_{w}R²; aryl-OCH₂CH(R⁵)CH₂R²; or heteroaryl-OCH₂CH(R⁵)CH₂R²;
R¹' and R¹' independently represent hydrogen; lower alkyl; lower alkenyl; lower alkinyl; cycloalkyl; aryl; or cycloalkyl - lower alkyl;
R² represents -OH, lower alkoxy, -OCOR³, -COOR³, -NR³R^{3'}, -OCONR³R³', -NCONR³R³', cyano, -CONR³R³', SO₃H, -SONR³R³', -CO-morpholin-4-yl, -CO-((4-lower alkyl)piperazin-1-yl), -NH(NH)NH₂, or -NR⁴R^{4'}, with the proviso that a carbon atom is attached at the most to one heteroatom in case this carbon atom is sp³-hybridized;
R³ and R³' independently represent hydrogen; lower alkyl; lower alkenyl; cycloalkyl; or cycloalkyl - lower alkyl;
R⁴ and R⁴' independently represent hydrogen; lower alkyl; cycloalkyl; cycloalkyl - lower alkyl; hydroxy - lower alkyl; -COOR²; or -CONH₂;
R⁵ represents -OH, -OR²; -OCOR²; or -OCOOR²; or R⁵ and R² form together with the carbon atoms to which they are attached a 1,3-dioxolane ring which is substituted in position 2 with R³ and R³'; or R⁵ and R² form together with the carbon atoms to which they are attached a 1,3-dioxolan-2-one ring;
p is the integer 1, 2, 3 or 4;
r is the integer 3, 4, 5 or 6;
s is the integer 2, 3, 4 or 5;
t is the integer 1, 2, 3 or 4;
u is the integer 1, 2 or 3;
v is the integer 2, 3 or 4;
w is the integer I or 2;
and optically pure enantiomers, mixtures of enantiomers such as racemates, diastereomers, mixtures of diastereomers, diastereomeric racemates, mixtures of diastereomeric racemates, and the meso-form; as well as pharmaceutically acceptable salts, solvent complexes and morphological forms.

In the definitions of general formula **I** - if not otherwise stated - the term **lower alkyl**, alone or in combination with other groups, means saturated, straight and branched chain groups with one to seven carbon atoms, preferably one to four carbon atoms that can be optionally substituted by halogens. Examples of lower alkyl groups are methyl, ethyl, n-propyl, iso-propyl, n-butyl, iso-butyl, sec-butyl, tert-butyl, pentyl, hexyl and heptyl. The methyl, ethyl and isopropyl groups are preferred.

The term **lower alkoxy** refers to a R-O-group, wherein R is a lower alkyl. Examples of lower alkoxy groups are methoxy, ethoxy, propoxy, iso-propoxy, iso-butoxy, sec-butoxy and tert-butoxy.

The term lower **alkenyl**, alone or in combination with other groups, means straight and branched chain groups comprising an olefinic bond and two to seven carbon atoms, preferably two to four carbon atoms, that can be optionally substituted by halogens. Examples of lower alkenyl are vinyl, propenyl or butenyl.

The term **lower alkinyl**, alone or in combination with other groups, means straight and branched chain groups comprising a triple bond and two to seven carbon atoms, preferably two to four carbon atoms, that can be optionally substituted by halogens. Examples of lower alkinyl are ethinyl, propinyl or butinyl.

The term **lower alkylene**, alone or in combination with other groups, means straight and branched divalent chain groups with one to seven carbon atoms, preferably one to four carbon atoms, that can be optionally substituted by halogens. Examples of lower alkylene are ethylene, propylene or butylene.

The term **lower alkenylene**, alone or in combination with other groups, means straight and branched divalent chain groups comprising an olefinic bond and two to seven carbon atoms, preferably two to four carbon atoms, that can be optionally substituted by halogens. Examples of lower alkenylene are vinylene, propenylene and butenylene.

The term **lower alkylenedioxy**, refers to a lower alkylene substituted at each end by an oxygen atom. Examples of lower alkylenedioxy groups are preferably methylenedioxy and ethylenedioxy.

The term **lower alkylenoxy** refers to a lower alkylene substituted at one end by an oxygen atom. Examples of lower alkylenoxy groups are preferably ethylenoxy and propylenoxy.

The term **halogen** means fluorine, chlorine, bromine or iodine, preferably fluorine, chlorine and bromine.

The term **cycloalkyl** alone or in combination, means a saturated cyclic hydrocarbon ring system with 3 to 7 carbon atoms, e.g. cyclopropyl, cyclobutyl, cyclopentyl, cyclohexyl and cycloheptyl, which can be optionally mono-, di-, or trisubstituted independently by lower alkyl, lower alkenyl, lower alkenylene, lower alkoxy, lower alkylenoxy, lower alkylenedioxy, hydroxy, halogen, -CF₃, -NR¹R¹', -NR¹C(O)R¹', -NR'S(O)₂R¹', -C(O)NR¹R¹', lower alkylcarbonyl,-COOR¹, -SR¹, -SOR¹, -SO₂R¹, -SO₂NR¹R¹'. The cyclopropyl group is a preferred group.

The term **aryl,** alone or in combination, relates to the phenyl, the naphthyl or the indanyl group, preferably the phenyl group, which can be optionally mono-, di-, tri-, tetra- or pentasubstituted independently by lower alkyl, lower alkenyl, lower alkinyl, lower alkenylene or lower alkylene forming with the aryl ring a five- or six-membered ring, lower alkoxy, lower alkylenedioxy, lower alkylenoxy, hydroxy, hydroxy-lower alkyl, halogen, cyano, -CF₃, -OCF₃, -NR¹R¹', -NR¹R¹'-lower alkyl, -NR¹C(O)R¹', -NR₁S(O)₂R¹', -C(O)NR¹R¹', -NO₂, lower alkylcarbonyl, -COOR¹, -SR¹, -S(O)R¹, -S(O)₂R¹, -SO₂NR¹R¹', benzyloxy. Preferred substituents are halogens, lower alkoxy, lower alkyl.

The term **heterocyclyl,** alone or in combination, means saturated or unsaturated (but not aromatic) five-, six- or seven-membered rings containing one or two nitrogen, oxygen or sulfur atoms which may be the same or different and which rings can be optionally substituted with lower alkyl, hydroxy, lower alkoxy and halogen. The nitrogen atoms, if present, can be substituted by a COOR² group. Examples of such rings are piperidinyl, morpholinyl, thiomorpholinyl, piperazinyl, tetrahydropyranyl, dihydropyranyl, 1,4-dioxanyl, pyrrolidinyl, tetrahydrofuranyl, dihydropyrrolyl, imidazolidinyl, dihydropyrazolyl, dihydroquinolinyl, tetrahydroquinolinyl, tetrahydroisoquinolinyl.

The term **heteroaryl,** alone or in combination, means six-membered aromatic rings containing one to four nitrogen atoms; benzofused six-membered aromatic rings containing one to three nitrogen atoms; five-membered aromatic rings containing one oxygen, one nitrogen or one sulfur atom; benzofused five-membered aromatic rings containing one oxygen, one nitrogen or one sulfur atom; five-membered aromatic rings containing one oxygen and one nitrogen atom and benzofused derivatives thereof; five-membered aromatic rings containing a sulfur and a nitrogen or an oxygen atom and benzofused derivatives thereof; five-membered aromatic rings containing two nitrogen atoms and benzofused derivatives thereof; five-membered aromatic rings containing three nitrogen atoms and benzofused derivatives thereof, or a tetrazolyl ring. Examples of such ring systems are furanyl, thiophenyl, pyrrolyl, pyridinyl, pyrimidinyl, indolyl, quinolinyl, isoquinolinyl, imidazolyl, triazinyl, thiazinyl, thiazolyl, isothiazolyl, pyridazinyl, pyrazolyl, oxazolyl, isoxazolyl, coumarinyl, benzothiophenyl, quinazolinyl, quinoxalinyl. Such rings may be adequatly substituted with lower alkyl, lower alkenyl, lower alkinyl, lower alkylene, lower alkenylene, lower alkylenedioxy, lower alkyleneoxy, hydroxy-lower alkyl, lower alkoxy, hydroxy, halogen, cyano, -CF₃, -OCF₃, -NR¹R¹', -NR¹R¹' - lower alkyl, -N(R')COR', -N(R¹)SO₂R¹, -CONR¹R¹', -NO₂, lower alkylcarbonyl, -COOR¹, -SR¹, -S(O)R¹, -S(O)₂R¹, -SO₂NR¹R¹', another aryl, another heteroaryl or another heterocyclyl and the like.

It is understoood that the substituents outlined relative to the expressions cycloalkyl, heterocyclyl, heteroaryl and aryl have been omitted in the definitions of the general formula I and in claims 1 to 5 for clarity reasons but the definitions in formula I and in claims 1 to 5 should be read as if they are included therein.

The expression **pharmaceutically acceptable** salts encompasses either salts with inorganic acids or organic acids like hydrochloric or hydrobromic acid, sulfuric acid, phosphoric acid, citric acid, formic acid, acetic acid, maleic acid, tartaric acid, benzoic acid, methanesulfonic acid, p-toluenesulfonic acid, and the like that are non toxic to living organisms or in case the compound of formula I is acidic in nature with an inorganic base like an alkali or earth alkali base, e.g. sodium hydroxide, potassium hydroxide, calcium hydroxide and the like.

The compounds of the general formula **I** can contain one or more asymmetric carbon atoms and may be prepared in form of optically pure enantiomers, mixtures of enantiomers such as racemates, diastereomers, mixtures of diastereomers, diastereomeric racemates, mixtures of diastereomeric racemates, and the meso-form and pharmaceutically acceptable salts thereof.

The present invention encompasses all these forms. Mixtures may be separated in a manner known *per se,* i.e. by column chromatography, thin layer chromatography, HPLC or crystallization.

A group of preferred compounds of general formula **I** are those wherein X, W, V, and U, are as defined in general formula **I** wherein
T is -CONR¹-;
Q is a methylene; and
M is aryl; heteroaryl; aryl-O(CH₂)ᵥR²; or heteroaryl-O(CH₂)ᵥR².

Another group of more preferred compounds of general formula **I** are those wherein X, W, T, Q, and M are as defined in general formula **I** wherein
V is one of the following groups:
-CH₂CH₂O-; -CH₂CH₂CH₂O-; or -OCH₂CH₂O-;
and U is as defined in general formula **I** above.

Another group of even more preferred compounds of general formula **I** are those wherein V, U, T, Q, and M are as defined in general formula **I** and wherein
X and W represent -CH-.

Another group of more preferred compounds of general formula **I** are those wherein X, W, V, Q, T, and M are as defined in general formula **I** and wherein
U is a mono-, di-, or trisubstituted phenyl or heteroaryl. Preferred substituents are independently halogen, lower alkyl, lower alkoxy, CF₃.

Especially preferred compounds of general formula I are those selected from the group consisting of:
4-{4-[3-(2,3,6-trifluorophenoxy)propyl]phenyl}-1,2,3,6-tetrahydropyridine-3-carboxylic acid cyclopropyl-(2-fluorobenzyl)amide;
4-{4-[3-(2,3,6-trifluorophenoxy)propyl]phenyl}-1,2,3,6-tetrahydropyridine-3-carboxylic acid (2-chlorobenzyl)ethylamide;
4- {4-[3-(2,3,6-trifluorophenoxy)propyl]phenyl} 1,2,3,6-tetrahydropyridine-3-carboxylic acid (2-chlorobenzyl)cyclopropylamide;
4-{4-[3-(2,3,6-trifluorophenoxy)propyl]phenyl}-1,2,3,6-tetrahydropyridine-3-carboxylic acid cyclopropyl-(2-fluoro-5-methoxybenzyl)amide;
4-{4-[3-(2,3,6-trifluorophenoxy)propyl]phenyl} -1,2,3,6-tetrahydropyridine-3-carboxylic acid cyclopropyl-(3-methoxybenzyl)amide;
4- {4-[3-(2,3,6-trifluorophenoxy)propyl]phenyl} -1,2,3,6-tetrahydropyridine-3-carboxylic acid cyclopropyl-(2-methoxybenzyl)amide;
4-{4-[3-(2,3,6-trifluorophenoxy)propyl]phenyl}-1,2,3,6-tetrahydro-pyridine-3-carboxylic acid cyclopropyl-(5-fluoro-2-methoxybenzyl)amide;
4-{4-[3-(2,3,6-trifluorophenoxy)propyl]phenyl}-1,2,3,6-tetrahydropyridine-3-carboxylic acid (2-chloro-6-fluorobenzyl)cyclopropylamide;
4-{4-[3-(2,3,6-trifluorophenoxy)propyl]phenyl}-1,2,3,6-tetrahydropyridine-3-carboxylic acid (2-bromobenzyl)cyclopropylamide;
4-{4-[3-(2,3,6-trifluorophenoxy)propyl]phenyl}-1,2,3,6-tetrahydropyridine-3-carboxylic acid cyclopropyl-(2,3-dimethylbenzyl)amide;
4- {4-[3-(2,3,6-trifluorophenoxy)propyl]-henyl}-1,2,3,6-tetrahydro-pyridine-3-carboxylic acid (3,5-bis-trifluoromethylbenzyl)cyclopropylamide;
4-{4-[3-(2,3,6-trifluorophenoxy)propyl]phenyl}-1,2,3,6-tetrahydropyridine-3-carboxylic acid (2-chloro-5-trifluoromethylbenzyl)cyclopropylamide;
4-{4-[3-(2,3,6-trifluorophenoxy)propyl]phenyl}-1,2,3,6-tetrahydropyridine-3-carboxylic acid (2-chloro-3,6-difluorobenzyl)cyclopropylamide; and
4-{4-[3-(2,3,6-trifluorophenoxy)propyl]phenyl}-1,2,3,6-tetrahydropyridine-3-carboxylic acid cyclopropyl-(3-methylbenzyl)amide.

The compounds of general formula I and their pharmaceutically acceptable salts may be used as therapeutics e.g. in form of pharmaceutical compositions. These pharmaceutical compositions containing at least one compound of general formula I and usual carrier materials and adjuvants may especially be used in the treatment or prophylaxis of disorders which are associated with a dysregulation of the renin angiotensin system (RAS), comprising cardiovascular and renal diseases. Examples of such diseases are hypertension, congestive heart failure, pulmonary heart failure, coronary diseases, cardiac insufficiency, renal insufficiency, renal or myocardial ischemia, atherosclerosis, and renal failure. They can also be used to prevent restenosis after balloon or stent angioplasty, to treat erectile dysfunction, glomerulonephritis, renal colic, and glaucoma. Furthermore, they can be used in the therapy and the prophylaxis of diabetic complications, complications after vascular or cardiac surgery, complications of treatment with immunosuppresive agents after organ transplantation, complications of cyclosporin treatment, as well as other diseases presently known to be related to the RAS.

In another embodiment, the invention relates to the use of a compound of formula I for the preparation of a pharmaceutical composition for the treatment and/or prophylaxis of diseases which are related to the RAS such as hypertension, congestive heart failure, pulmonary hypertension, cardiac insufficiency, renal insufficiency, renal or myocardial ischemia, atherosclerosis, renal failure, erectile dysfunction, glomerulonephritis, renal colic, glaucoma, diabetic complications, complications after vascular or cardiac surgery, restenosis, complications of treatment with immunosuppresive agents after organ transplantation, and other diseases which are related to the RAS.

The compounds of formula **I** may also be used in combination with one or more other pharmacologically active compounds e. g. with other renin inhibitors, with ACE-inhibitors, angiotensin II receptor antagonists, endothelin receptor antagonists, vasodilators, calcium antagonists, potassium activators, diuretics, sympatholitics, beta-adrenergic antagonists, and neutral endopeptidase inhibitors, alpha-adrenergic antagonists, for the treatment of disorders as above-mentioned.

The compounds of general formula **I** can be manufactured by the methods given below, by the methods given in the examples or by analogous methods.

### Preparation of the precursors:

Precursors are compounds that were prepared as key intermediates and/or building blocks and which were suitable for further transformations in parallel chemistry.

Ideal starting materials are any commercially available 4-oxo-piperidine-3-carboxylic acid ester derivatives, for instance 1-benzyl-4-oxo-piperidine-3-carboxylic acid methyl ester, possibly as a salt. For practical purposes, a transesterification (for instance according to Seebach D., et al., Synthesis, 1982, 138) to another ester derivative **A** (wherein R^{a} is optionally a lower alkyl, a lower alkenyl, or a benzyl group), thereafter a change in the *N*-protecting group (PG: all abreviations are outlined at the beginning of the chapter Examples) to a derivative of type **B,** may be necessary (Scheme 1).

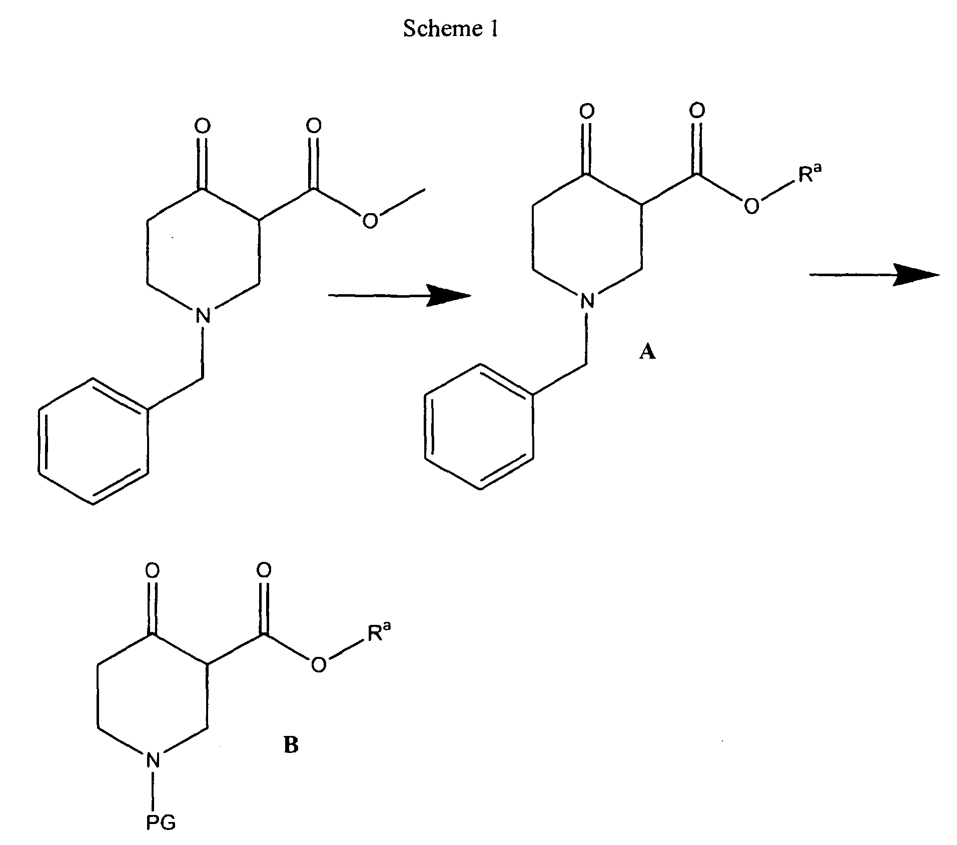

Formation of the vinyl triflate **C**, followed by a coupling catalysed by a Pd(O) complex leads to tetrahydropyridine derivatives of type **D**, wherein R^{b} optionally represents any U-V group as defined in general formula **I** or a chemical precursor of such a group (Scheme 2).

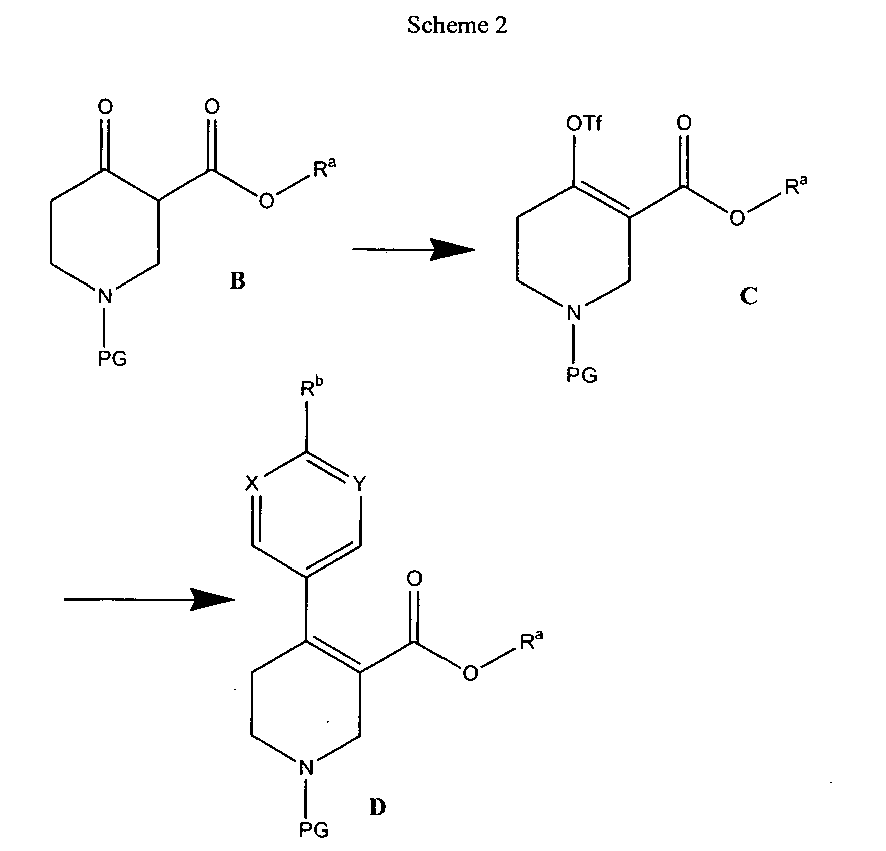

If, for instance, R^{b} is a linker ending with a silanyl ether, compounds of type **D** can be deprotected to compounds of type **E**, then coupled to a phenol or aromatic alcohol using a *Mitsunobu* reaction, leading to derivatives of type **F** wherein V and U have the meaning given in general formula **I** above (Scheme 3). The ester **F** can then be cleaved under basic conditions to lead to precursor **G**, whereas the double bond would partially or completely be shifted to the 4,5-position.

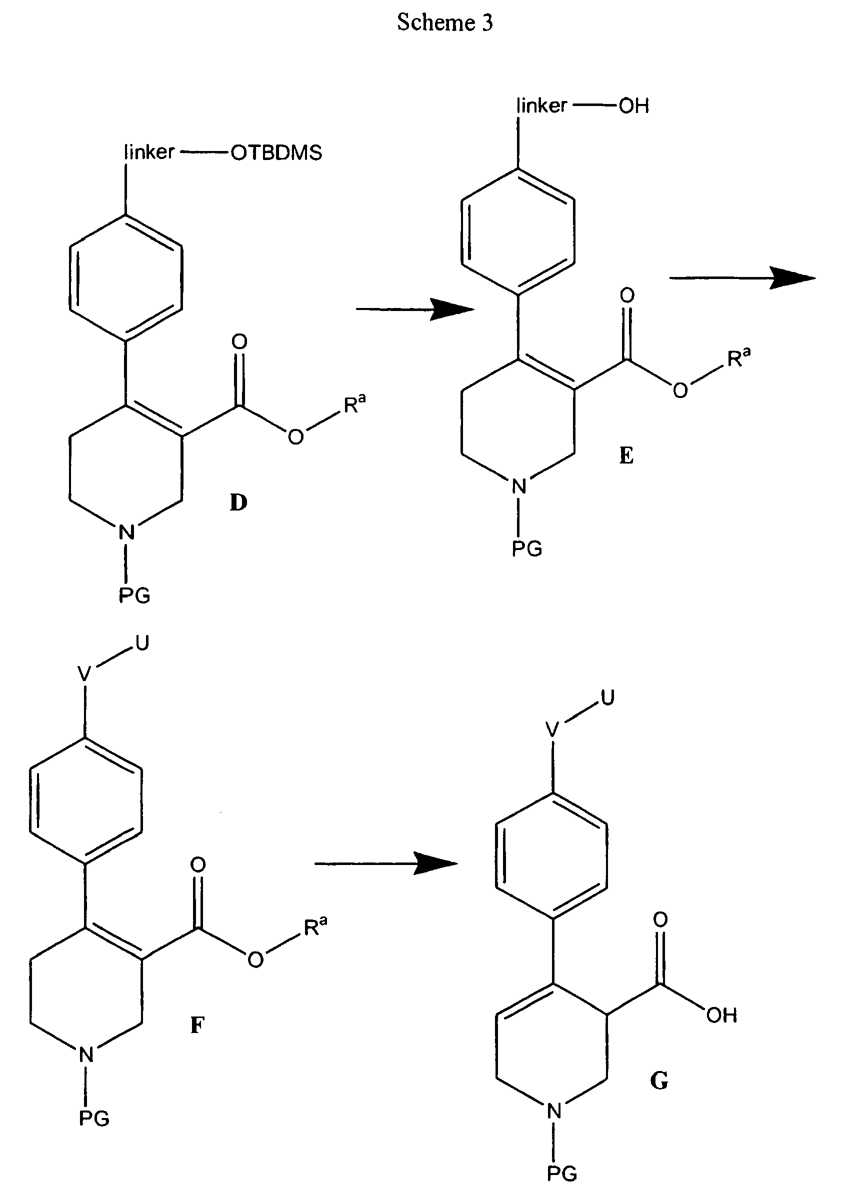

Other chemistry may be used in analogy to patent applications WO03/093267 and WO04/002957. This allows to prepare other compounds included in general formula **I**.

### Preparation of final compounds

A compound of type **G** can be coupled to the amine to yield to the corresponding amides wherein V, U and M have the meaning given in general formula **I** above. Removal of the *N*-protecting group (PG) leads to the final compounds wherein V, U, Q and M have the meaning given in general formula **I** above (Scheme 4). If the precursor **G** is mixed to the corresponding constitutional isomer having a double bond at the 3,4-position, both compounds may be now separated by flash chromatography, using some ammoniac as co-eluent, or by HPLC.

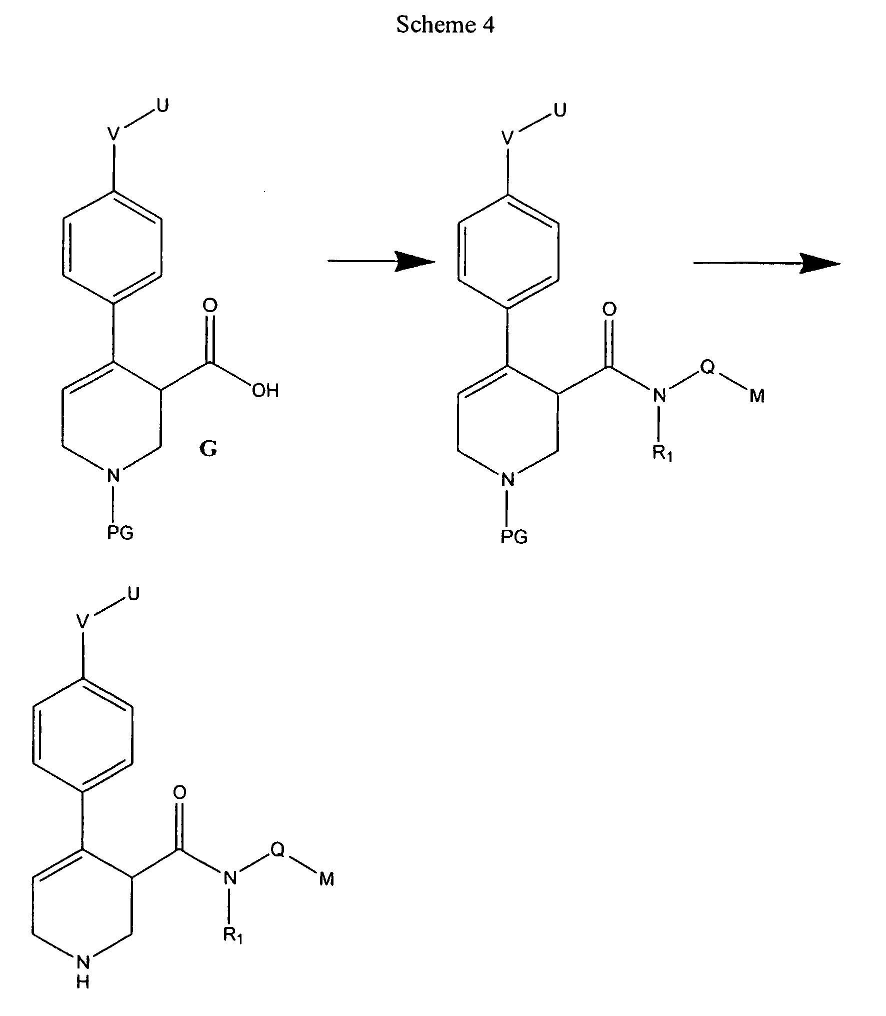

The compounds of formula I and their pharmaceutically acceptable acid addition salts can be used as medicaments, e. g. in the form of pharmaceutical preparations for enteral, parenteral, or topical administration. They can be administered, for example, perorally, e. g. in the form of tablets, coated tablets, dragées, hard and soft gelatine capsules, solutions, emulsions or suspensions, rectally, e. g. in the form of suppositories, parenterally, e. g. in the form of injection solutions or infusion solutions, or topically, e. g. in the form of ointments, creams or oils.

The production of pharmaceutical preparations can be effected in a manner which will be familiar to any person skilled in the art by bringing the described compounds of formula **I** and their pharmaceutically acceptable acid addition salts, optionally in combination with other therapeutically valuable substances, into a galenical administration form together with suitable, non-toxic, inert, therapeutically compatible solid or liquid carrier materials and, if desired, usual pharmaceutical adjuvants in a manner known per se.

Suitable carrier materials are not only inorganic carrier materials, but also organic carrier materials. Thus, for example, lactose, corn starch or derivatives thereof, talc, stearic acid or its salts can be used as carrier materials for tablets, coated tablets, dragées and hard gelatine capsules. Suitable carrier materials for soft gelatine capsules are, for example, vegetable oils, waxes, fats and semi-solid and liquid polyols (depending on the nature of the active ingredient no carriers are, however, required in the case of soft gelatine capsules). Suitable carrier materials for the production of solutions and syrups are, for example, water, polyols, sucrose, invert sugar and the like. Suitable carrier materials for injections are, for example, water, alcohols, polyols, glycerols and vegetable oils. Suitable carrier materials for suppositories are, for example, natural or hardened oils, waxes, fats and semi-liquid or liquid polyols. Suitable carrier materials for topical preparations are glycerides, semi-synthetic and synthetic glycerides, hydrogenated oils, liquid waxes, liquid paraffins, liquid fatty alcohols, sterols, polyethylene glycols and cellulose derivatives.

Usual stabilizers, preservatives, wetting and emulsifying agents, consistency-improving agents, flavour-improving agents, salts for varying the osmotic pressure, buffer substances, solubilizers, colorants and masking agents and antioxidants come into consideration as pharmaceutical adjuvants.

The dosage of compounds of formula **I** can vary within wide limits depending on the disease to be controlled, the age and the individual condition of the patient and the mode of administration, and will, of course, be fitted to the individual requirements in each particular case. For adult patients a daily dosage of about 1 mg to about 1000 mg, especially about 50 mg to about 500 mg, comes into consideration. For children the dosage has to be adapted to the body weight and age.

The pharmaceutical preparations conveniently contain about 1 - 500 mg, preferably 5 - 200 mg of a compound of formula **I**.

The following examples serve to illustrate the present invention in more detail. They are, however, not intended to limit its scope in any manner.

### Examples

### General remarks

The following compounds were prepared according to the procedures described for the synthesis of compounds encompassed by the general formula **I**. All compounds were characterized by ¹H-NMR (300 MHz) and occasionally by ¹³C-NMR (75 MHz) (Varian Oxford, 300 MHz; chemical shifts are given in ppm relative to TMS), by LC-MS: **A**: 2 min < t_{R} < 10 min; (Waters Micromass; ZMD-platform with ESI-probe with Alliance 2790 HT; Column: 2x30 mm, Gromsil ODS4, 3 µM, 120A; Gradient: 0 - 100% acetonitril in water, 6 min, with 0.05% formic acid, flow: 0.45 mL/min; t_{R} given in min.), **B**: 0.1 min < t_{R} < 2 min; (Finnigan AQA with ESI-probe with HP 110 DAD and HP110 binary pump; column: Develosil RP-AQUEOUS, 5 µM, 4.6 mm x 50 mm; gradient: 5 - 95% methanol in water (0.04% TFA), I min, 95% methanol in water (0.04% TFA) 0.4 min, 4.5 mL/min.), by TLC (TLC-plates from Merck, Silica gel 60 F₂₅₄). Only TLC and LC-MS data are given hereby.

### Abbreviations

- ACE: Angiotensin Converting Enzyme
- Ang: Angiotensin
- aq.: aqueous
- Bn: Benzyl
- Boc: tert-Butyloxycarbonyl
- BSA: Bovine serum albumine
- BuLi: n-Butyllithium
- DIPEA: Diisopropylethylamine
- DMAP: 4-*N,N*-Dimethylaminopyridine
- DMSO: Dimethylsulfoxide
- EDC HCl: Ethyl-*N,N*-dimethylaminopropylcarbodiimide hydrochloride
- EIA: Enzyme immunoassay
- eq.: equivalent
- Et: Ethyl
- EtOAc: Ethyl acetate
- FC: Flash Chromatography
- HOBt: Hydroxybenzotriazol
- MeOH: Methanol
- org.: organic
- PBS: Phosphate Buffer Saline
- PG: protecting group
- Ph: Phenyl
- RAS: Renin Angiotensin System
- RP18: Reversed phase column, filled with C₁₈ hydrocarbon
- rt: room temperature
- sol.: Solution
- TBDMS: tert-Butyldimethylsilyl
- Tf: Trifluoromethylsulfonyl
- TFA: Trifluoroacetic acid
- THF: Tetrahydrofuran
- TLC: Thin Layer Chromatography

### Preparation of the precursors

### 4-Oxopiperidine-1,3-dicarboxylic acid 1-tert-butyl ester 3-methyl ester (B)

A suspension of 1-benzyl-4-oxopiperidine-3-carboxylic acid methyl ester hydrochloride (5.00 g, 17.6 mmol), triethylamine (2.45 mL, 17.6 mmol) and Boc₂O (4.20 g, 20.0 mmol) in EtOH (30 mL) was purged with N₂. Pd/C (10%, 600 mg) was added and the suspension purged with H₂. The reaction mixture was stirred under an H₂-atmosphere for 24 h and then filtered through *Celite.* The filtrate was evaporated under reduced pressure. Purification of the residue by FC (EtOAc/heptane 1:4 → 2:3) yielded the title compound (4.02 g, 89%). R_{f} = 0.60 (EtOAc/heptane 1:1). LC-MS: Rₜ = 1.09 min, ES+ = 202.03.

### 4-Trifluoromethanesulfonyloxy-5,6-dihydro-2H-pyridine-1,3-dicarboxylic acid 1-tert-butyl ester 3-methyl ester (C)

To a sol. of compound **B** (4.00 g, 15.6 mmol) in THF (100 mL) at 0 °C was added NaH (suspension in oil, 55-65%, 1.20 g, about 31 mmol). The suspension was stirred for 30 min at 0 °C and Tf₂NPh (8.27 g, 23.1 mmol) was added. The ice bath was removed and the reaction mixture stirred for 3 days at rt. Ice was added and the solvents were removed under reduced pressure. The residue was diluted with EtOAc and washed with aq. 10% Na₂CO₃. The org. extracts were dried over MgSO₄, filtered and the solvent removed under reduced pressure. Purification of the residue by FC (EtOAc/heptane 1:4) yielded the title compound (5.19 g, 86%). LC-MS: Rₜ = 1.17, ES+ = 374.96.

### 4-{4-[3-(tert-Butyldimethylsilanyloxy)propyl] phenyl}-5,6-dihydro-2H-pyridine-1,3-dicarboxylic acid 1-tert-butyl ester 3-methyl ester (D)

To a sol. of [3-(4-bromophenyl)propoxy]-*tert*-butyldimethylsilane (Kiesewetter D. O., Tetrahedron Asymmetry, 1993, 4, 2183; 6.19 g, 19.7 mmol) in THF (100 mL) at -78 °C was added n-BuLi (1.5M in hexane, 14.0 mL, 21.0 mmol). The sol. was stirred at -78 °C for 30 min and ZnCl₂ (1M in THF, 22.3 mL, 22.3 mmol) was added. The resulting sol. was allowed to warm to rt and compound C (5.10 g, 13.1 mmol) and Pd(PPh₃)₄ (300 mg, 0.26 mmol) were added. After 20 min at rt ice was added to the reaction mixture. The solvents were removed under reduced pressure and the residue diluted with EtOAc. This mixture was washed with aq. 1M NaOH. The org. extracts were dried over MgSO₄, filtered and the solvents removed under reduced pressure. Purification of the residue by FC (EtOAc/heptane 1:9) led to the title compound (5.77 g, 90%). LC-MS: Rₜ = 7.27 min, ES+ = 512.54.

### 4-[4-(3-Hydroxypropyl)phenyl]-5,6-dihydro-2H-pyridine-1,3-dicarboxylic acid 1-tert-butyl ester 3-methyl ester (E)

TBAF (1.90 g, 6.00 mmol) was added to a sol. of compound **D** (1.95 g, 4.00 mmol) in THF (40 mL). The reaction mixture was stirred for 6 h at rt and diluted with EtOAc. The resulting mixture was washed with water and brine. The org. extracts were dried over MgSO₄, filtered and the solvents removed under reduced pressure. Purification of the residue by FC (EtOAc/heptane 2:3) yielded the title compound (1.27 g, 84%). LC-MS: Rₜ = 1.06, ES+ = 376.18.

### 4-{4-[3-(2,3,6-Trifluorophenoxy)propyl]phenyl}-5,6-dihydro-2H-pyridine-1,3-dicarboxylic acid 1-tert-butyl ester 3-methyl ester (F)

A sol. of compound **E** (4.7 g, 12.5 mmol), 2,3,6-trifluorophenol (3.7 g, 25.0 mmol), azodicarboxylic dipiperidine (6.32 g, 34.2 mmol), tri-n-butylphosphine (85%, 9.3 mL, 37.6 mmol) and DIPEA (0.035 mL, 0.20 mmoL) in toluene (20 mL) was stirred for 1 h at rt, then for 2 h at 60 °C. The reaction mixture was allowed to cool to rt, was diluted with EtOAc and washed with water. The org. extracts were dried over MgSO₄, filtered and the solvents were removed under reduced pressure. Purification of the residue by FC (EtOAc/heptane 1:4 → 3:7) led to the title compound (5.23 g, 83%).

### 4-{4-[3-(2,3,6-Trifluorophenoxy)propyl]phenyl}-1,2-dihydro-5H-pyridine-1,3-dicarboxylic acid 1-tert-butyl ester (G)

To a sol. of compound **F** (5.23 g, 10.3 mmol) in EtOH (90 mL) was added aq. 1M NaOH (90 mL). The resulting mixture was stirred for 35 min at 80 °C, then allowed to cool to rt. Aq. 1M HCl (13 mL) was added and the resulting mixture was extracted with EtOAc (3x). The combined org. extracts were dried over MgSO₄, filtered and the solvents were removed under reduced pressure. Purification of the residue by FC (EtOAc/heptane 2:3) led to the title compound (4.55 g, 89%).

### Preparation of the final compounds

### General procedure A for amide coupling

A sol. of the desired carboxylic acid (1.00 eq), the desired amine (2.00 eq), EDC·HCl (1.10 eq.), HOBt (cat. amount), DMAP (cat. amount) and DIPEA (2.00 eq.) in CH₂Cl₂ (20 mL/g of acid) was stirred at rt overnight. The reaction mixture was washed over diatomic earth (Isolute Sorbent Technology, Johnson, C. R., et al., Tetrahedron, 1998, 54, 4097) and the org. extracts were evaporated under reduced pressure. The residue was used without further purification.

### General procedure B for the removal of a Boc-protecting group

The starting material was dissolved in CH₂Cl₂ (10 mL/g of starting material) and the sol. was cooled to 0 °C. 4M HCl in dioxane (same volume as CH₂Cl₂) was added and the reaction mixture was left for 90 min at rt. The solvents were removed under reduced pressure. Purification of the residue by HPLC led to the desired compound.

### General procedure C for reductive amination

To a solution of aldehyde (1eq.) in MeOH (0.5 mL/mmol) was added an amine (1.2 eq.). The solution was stirred for 2h. Sodium borohydride (1.2 eq.) was added portionwise at 0°C and then stirring was continued, at rt, for 4h. A sol. of aq. 1M NaOH was added and the MeOH was evaporated. The mixture was extracted with EtOAc twice and the organic layer was washed with brine, dried over Na₂SO₄ and filtered. The solvent was removed under reduced pressure. The isolated amines were either used without further purification or purified by flash chromatography (EtOAc/heptane: 2/8), depending on the purity.

### Preparation of the secondary amines

### Cyclopropyl(2-fluorobenzyl)amine

Synthesized according to general procedure C from 2-fluorobenzaldehyde and cyclopropylamine.

### (2-Chlorobenzyl)cyclopropylamine

Synthesized according to general procedure C from 2-chlorobenzaldehyde and cyclopropylamine.

### (2-Chlorobenzyl)ethylamine

See Ishihara, Y; et al.; Chem. Pharm. Bull., 1991, 39, 3225*.*

### Cyclopropyl-(2-fluoro-5-methoxybenzyl)amine

Synthesized according to general procedure C from 2-fluoro-5-methoxybenzaldehyde and cyclopropylamine.

### Cyclopropyl-(3-methoxybenzyl)amine

Synthesized according to general procedure C from 3-methoxybenzaldehyde and cyclopropylamine.

### Cyclopropyl-(2-methoxybenzyl)amine

Synthesized according to general procedure C from 2-methoxybenzaldehyde and cyclopropylamine.

### Cyclopropyl-(5-fluoro-2-methoxybenzyl)amine

Synthesized according to general procedure C from 5-fluoro-2-methoxybenzaldehyde and cyclopropylamine.

### (2-Chloro-6-fluorobenzyl)cyclopropylamine

Synthesized according to general procedure C from 2-chloro-6-fluorobenzaldehyde and cyclopropylamine.

### (2-Bromobenzyl)cyclopropylamine

Synthesized according to general procedure C from 2-bromobenzaldehyde and cyclopropylamine.

### Cyclopropyl-(2,3-dimethylbenzyl)amine

Synthesized according to general procedure C from 2,3-dimethylbenzaldehyde and cyclopropylamine.

### (3,5-Bistrifluoromethylbenzyl)cyclopropylamine

Synthesized according to general procedure C from 3,5-bistrifluoromethylbenzaldehyde and cyclopropylamine.

### (2-Chloro-3,6-difluorobenzyl)cyclopropylamine

Synthesized according to general procedure C from 2-chloro-3,6-difluorobenzaldehyde and cyclopropylamine.

### Cyclopropyl-(3-methylbenzyl)amine

Synthesized according to general procedure C from 3-methylbenzaldehyde and cyclopropylamine.

### Examples:

### Example 1

### (rac.)-4-{4-[3-(2,3,6-Trifluorophenoxy)propyl]phenyl}-1,2,3,6-tetrahydropyridine-3-carboxylic acid cyclopropyl-(2-fluorobenzyl)amide

Prepared according to the general procedures A and B with cyclopropyl-(2-fluorobenzyl)amine. LC-MS: Rₜ = 0.93; ES+: 539.21.

### Example 2

### (rac.)-4-{4-[3-(2,3,6-Trifluorophenoxy)propyl]phenyl}-1,2,3,6-tetrahydropyridine-3-carboxylic acid (2-chlorobenzyl)ethylamide

Prepared according to the general procedures A and B with (2-chlorobenzyl)ethylamine. LC-MS: Rₜ = 0.94; ES+: 543.17.

### Example 3

### (rac.)-4-{4-[3-(2,3,6-Trifluorophenoxy)propyl)phenyl}-1,2,3,6-tetrahydropyridine-3-carboxylic acid (2-chlorobenzyl)cyclopropylamide

Prepared according to the general procedures A and B with (2-chlorobenzyl)cyclopropylamine. LC-MS: Rₜ = 0.94; ES+: 555.19.

### Example 4

### (rac.)-4-{4-(3-(2,3,6-Trifluorophenoxy)propyl]phenyl}-1,2,3,6-tetrahydropyridine-3-carboxylic acid cyclopropyl-(2-fluoro-5-methoxybenzyl)amide formate salt

Prepared according to the general procedures A and B with cyclopropyl (2-fluoro-5-methoxybenzyl)amine. LC-MS: Rₜ = 0.94; ES+: 569.14.

### Example 5

### (rac.)-4-{4-[3-(2,3,6-Trifluorophenoxy)propyl]phenyl}-1,2,3,6-tetrahydropyridine-3-carboxylic acid cyclopropyl-(3-methoxybenzyl)amide formate salt

Prepared according to the general procedures A and B with cyclopropyl (3-methoxybenzyl)amine. LC-MS: Rₜ = 0.93; ES+: 551.18.

### Example 6

### (rac.)-4-{4-[3-(2,3,6-Trifluorophenoxy)propyl]phenyl}-1,2,3,6-tetrahydropyridine-3-carboxylic acid cyclopropyl-(2-methoxybenzyl)amide formate salt

Prepared according to the general procedures A and B with cyclopropyl (2-methoxybenzyl)amine. LC-MS: Rₜ = 0.94; ES+: 551.18.

### Example 7

### (rac.)-4-{4-(3-(2,3,6-Trifluorophenoxy)propyl]phenyl}-1,2,3,6-tetrahydropyridine-3-carboxylic acid cyctopropyl-(5-fluoro-2-methoxybenzyl)amide formate salt

Prepared according to the general procedures A and B with cyclopropyl (5-fluoro-2-methoxybenzyl)amine. LC-MS: Rₜ = 0.95; ES+: 569.15.

### Example 8

### (rac.)-4-{4-[3-(2,3,6-Trifluorophenoxy)propyl]phenyl}-1,2,3,6-tetrahydropyridine-3-carboxylic acid (2-chloro-6-fluorobenzyl)cyclopropylamide formate salt

Prepared according to the general procedures A and B with (2-chloro-6-fluorobenzyl)cyclopropylamine. LC-MS: Rₜ = 0.95; ES+: 573.10.

### Example 9

### (rac.)-4-{4-[3-(2,3,6-Trifluorophenoxy)propyl]phenyl}-1,2,3,6-tetrahydropyridine-3-carboxylic acid (2-bromobenzyl)cyclopropylamide formate salt

Prepared according to the general procedures A and B with (2-bromobenzyl)cyclopropylamine. LC-MS: Rₜ = 0.96; ES+: 599.04.

### Example 10

### (rac.)-4-{4-[3-(2,3,6-Trifluorophenoxy)propyl)phenyl}-1,2,3,6-tetrahydropyridine-3-carboxylic acid cyclopropyl-(2,3-dimethylbenzyl)amide formate salt

Prepared according to the general procedures A and B with cyclorpopyl-(2,3-dimethylbenzyl)amine. LC-MS: Rₜ = 0.97; ES+: 549.17.

### Example 11

### (rac.)-4-{4-[3-(2,3,6-Trifluorophenoxy)propyl}-henyl}-1,2,3,6-tetrahydropyridine-3-carboxylic acid (3,5-bistrifluoromethylbenzyl)cyclopropylamide formate salt

Prepared according to the general procedures A and B with (3,5-bistrifluoromethylbenzyl)cyclopropylamine. LC-MS: Rₜ = 1.00; ES+: 657.13.

### Example 12

### (rac.)-4-{4-[3-(2,3,6-Trifluorophenoxy)propyl]phenyl}-1,2,3,6-tetrahydropyridine-3-carboxylic acid (2-chloro-3,6-difluorobenzyl)cyclopropylamideformate salt

Prepared according to the general procedures A and B with (2-chloro-3,6-difluorobenzyl)cyclopropylamine. LC-MS: Rₜ = 0.96; ES+: 591.12.

### Example 13

### (rac.)-4-{4-(3-(2,3,6-Trifluorophenoxy)propyl]phenyl}-1,2,3,6-tetrahydropyridine-3-carboxylic acid cyclopropyl-(3-methylbenzyl)amide formate salt

Prepared according to the general procedures A and B with cyclopropyl-(3-methylbenzyl)amine. LC-MS: Rₜ = 0.95; ES+: 535.19.

The following assay was carried out in order to determine the activity of the compounds of general formula **I** and their salts.

### Inhibition of human recombinant renin by the compounds of the invention

The enzymatic in vitro assay was performed in 384-well polypropylene plates (Nunc). The assay buffer consisted of 10 mM PBS (Gibco BRL) including I mM EDTA and 0.1 % BSA. The incubates were composed of 50 µL per well of an enzyme mix and 2.5 µL of renin inhibitors in DMSO. The enzyme mix was premixed at 4°C and consists of the following components:
- human recombinant renin (0.16 ng/mL)
- synthetic human angiotensin(1-14) (0.5 µM)
- hydroxyquinoline sulfate (1 mM)
The mixtures were then incubated at 37°C for 3 h.
To determine the enzymatic activity and its inhibition, the accumulated Ang I was detected by an enzyme immunoassay (EIA) in 384-well plates (Nunc). 5 µL of the incubates or standards were transferred to immuno plates which were previously coated with a covalent complex of Ang I and bovine serum albumin (Ang I - BSA). 75 µL of Ang I-antibodies in assay buffer above including 0.01% Tween 20 were added and a primary incubation made at 4 °C overnight. The plates were washed 3 times with PBS including 0.01 % Tween 20, and then incubated for 2 h at rt with an antirabbit-peroxidase coupled antibody (WA 934, Amersham). After washing the plates 3 times, the peroxidase substrate ABTS (2.2'-azino-di-(3-ethylbenzthiazolinsulfonate), was added and the plates incubated for 60 min at rt. After stopping the reaction with 0.1 M citric acid pH 4.3 the plate was evaluated in a microplate reader at 405 nm. The percentage of inhibition was calculated of each concentration point and the concentration of renin inhibition was determined that inhibited the enzyme activity by 50% (IC₅₀). The IC₅₀-values of all compounds tested are below 1 µM. Selected compounds exhibit a very good bioavailibility and are metabolically more stable than prior art compounds.

## Claims

1. Compounds of the general formula I 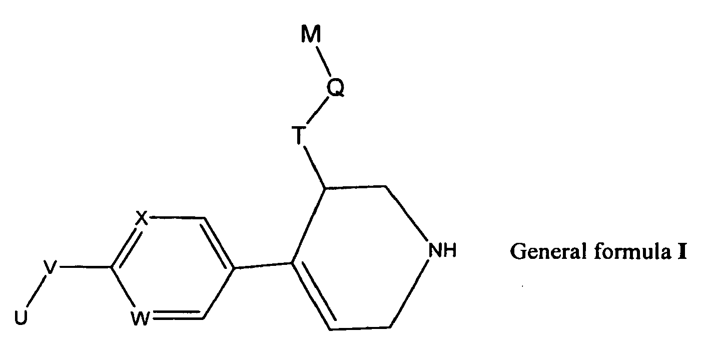 wherein
X and W represent independently a nitrogen atom or a CH-group;
V represents -(CH₂)ᵣ-; -A-(CH₂)ₛ-; -CH₂-A-(CH₂)ₜ-; -(CH₂)ₛ-A-; -(CH₂)₂-A-(CH₂)ᵤ-; -A-(CH₂)ᵥ-B-; -CH₂-CH₂-CH₂-A-CH₂-; -A-CH₂-CH₂-B-CH₂-; -CH₂-A-CH₂-CH₂-B-; -CH₂-CH₂-CH₂-A-CH₂-CH₂-; -CH₂-CH₂-CH₂-CH₂-A-CH₂-; -A-CH₂-CH₂-B-CH₂-CH₂-; -CH₂-A-CH₂-CH₂-B-CH₂-; -CH₂-A-CH₂-CH₂-CH₂-B-; or -CH₂-CH₂-A-CH₂-CH₂-B-;
A and B independently represent -O-; -S-; -SO-; or -SO₂-;
U represents aryl; or heteroaryl;
T represents -CONR¹-; -(CH₂)pOCO-; -(CH₂)ₚN(R¹)CO-; -(CH₂)ₚN(R¹)SO₂-; -COO-; -(CH₂)ₚOCONR¹-; or -(CH₂)ₚN(R¹')CONR¹-;
Q represents lower alkylene; or lower alkenylene;
M represents hydrogen; cycloalkyl; aryl; heterocyclyl; heteroaryl; aryl-O(CH₂)ᵥR²; heteroaryl-O(CH₂)ᵥR²; aryl-O(CH₂)₂O(CH₂)_{w}R²; heteroaryl-(CH₂)₂O(CH₂)_{w}R²; aryl-OCH₂CH(R⁵)CH₂R²; or heteroaryl-OCH₂CH(R⁵)CH₂R²;
R¹ and R¹' independently represent hydrogen; lower alkyl; lower alkenyl; lower alkinyl; cycloalkyl; aryl; or cycloalkyl - lower alkyl;
R² represents -OH, lower alkoxy, -OCOR³, -COOR³, -NR³R³', -OCONR³R³', -NCONR³R³', cyano, -CONR³R³', SO₃H, -SONR³R³', -CO-morpholin-4-yl, -CO-((4-lower alkyl)piperazin-1-yl), -NH(NH)NH₂, or -NR⁴R⁴', with the proviso that a carbon atom is attached at the most to one heteroatom in case this carbon atom is sp³-hybridized;
R³ and R³' independently represent hydrogen; lower alkyl; lower alkenyl; cycloalkyl; or cycloalkyl - lower alkyl;
R⁴ and R⁴' independently represent hydrogen; lower alkyl; cycloalkyl; cycloalkyl - lower alkyl; hydroxy - lower alkyl; -COOR²; or -CONH₂;
R⁵ represents -OH, -OR²; -OCOR²; or -OCOOR²; or R⁵ and R² form together with the carbon atoms to which they are attached a 1,3-dioxolane ring which is substituted in position 2 with R³ and R³'; or R⁵ and R² form together with the carbon atoms to which they are attached a 1,3-dioxolan-2-one ring;
p is the integer 1, 2, 3 or 4;
r is the integer 3, 4, 5, or 6;
s is the integer 2, 3, 4 or 5;
t is the integer 1, 2, 3 or 4;
u is the integer 1, 2 or 3;
v is the integer 2, 3 or 4;
w is the integer 1 or 2;
and wherein the prefix "lower" denotes a radical having up to 7 carbon atoms;
and optically pure enantiomers, mixtures of enantiomers such as racemates, diastereomers, mixtures of diastereomers, diastereomeric racemates, mixtures of diastereomeric racemates, and the meso-form; as well as pharmaceutically acceptable salts, solvent complexes and morphological forms.

2. Compounds of general formula **I** according to claim 1 wherein X, W, V, and U are as defined in claim 1, wherein
T represents -CONR¹-;
Q represents a methylene;
M represents aryl; heteroaryl; aryl-O(CH₂)ᵥR²; or heteroaryl-O(CH₂)ᵥR²;
and optically pure enantiomers, mixtures of enantiomers such as racemates, diastereomers, mixtures of diastereomers, diastereomeric racemates, mixtures of diastereomeric racemates, and the meso-form; as well as pharmaceutically acceptable salts, solvent complexes and morphological forms.

3. Compounds of general formula I according to claim 1 wherein X, W, T, Q, and M are as defined in claim 1, wherein
V represents
-CH₂CH₂O-; -CH₂CH₂CH₂O-; or -OCH₂CH₂O-;
and U is as defined in claim 1,
and optically pure enantiomers, mixtures of enantiomers such as racemates, diastereomers, mixtures of diastereomers, diastereomeric racemates, mixtures of diastereomeric racemates, and the meso-form; as well as pharmaceutically acceptable salts, solvent complexes and morphological forms.

4. Compounds of general formula **I** according to claim 1 wherein V, U, T, Q, and M are as defined in claim 1, wherein
X and W represent -CH-;
and optically pure enantiomers, mixtures of enantiomers such as racemates, diastereomers, mixtures of diastereomers, diastereomeric racemates, mixtures of diastereomeric racemates, and the meso-form; as well as pharmaceutically acceptable salts, solvent complexes and morphological forms.

5. Compounds of general formula **I** according to claim 1 wherein X, W, V, Q, T, and M are as defined in claim 1, wherein
U represents a mono-, di-, or trisubstituted phenyl or heteroaryl, wherein the substituents are independently halogen, lower alkyl, lower alkoxy, CF₃;
and optically pure enantiomers, mixtures of enantiomers such as racemates, diastereomers, mixtures of diastereomers, diastereomeric racemates, mixtures of diastereomeric racemates, and the meso-form; as well as pharmaceutically acceptable salts, solvent complexes and morphological forms.

6. The compounds according to any one of claims 1 to 5 selected from the group consisting of
4-{4-[3-(2,3,6-trifluorophenoxy)propyl]phenyl}-1,2,3,6-tetrahydropyridine-3-carboxylic acid cyclopropyl-(2-fluorobenzyl)amide;
4-{4-[3-(2,3,6-trifluorophenoxy)propyl]phenyl}-1,2,3,6-tetrahydropyridine-3-carboxylic acid (2-chlorobenzyl)ethylamide;
4-{4-[3-(2,3,6-trifluorophenoxy)propyl]phenyl}-1,2,3,6-tetrahydropyridine-3-carboxylic acid (2-chlorobenzyl)cyclopropylamide;
4-{4-[3-(2,3,6-trifluorophenoxy)propyl]phenyl}-1,2,3,6-tetrahydropyridine-3-carboxylic acid cyclopropyl-(2-fluoro-5-methoxybenzyl)amide;
4-{4-[3-(2,3,6-trifluorophenoxy)propyl]phenyl}-1,2,3,6-tetrahydropyridine-3-carboxylic acid cyclopropyl-(3-methoxybenzyl)amide;
4-{4-[3-(2,3,6-trifluorophenoxy)propyl]phenyl}-1,2,3,6-tetrahydropyridine-3-carboxylic acid cyclopropyl-(2-methoxybenzyl)amide;
4-{4-[3-(2,3,6-trifluorophenoxy)propyl]phenyl}-1,2,3,6-tetrahydro-pyridine-3-carboxylic acid cyclopropyl-(5-fluoro-2-methoxybenzyl)amide;
4-{4-[3-(2,3,6-trifluorophenoxy)propyl]phenyl}-1,2,3,6-tetrahydropyridine-3-carboxylic acid (2-chloro-6-fluorobenzyl)cyclopropylamide;
4-{4-[3-(2,3,6-trifluorophenoxy)propyl]phenyl}-1,2,3,6-tetrahydropyridine-3-carboxylic acid (2-bromobenzyl)cyclopropylamide;
4-{4-[3-(2,3,6-trifluorophenoxy)propyl]phenyl}-1,2,3,6-tetrahydropyridine-3-carboxylic acid cyclopropyl-(2,3-dimethylbenzyl)amide;
4-{4-[3-(2,3,6-trifluorophenoxy)propyl]-henyl}-1,2,3,6-tetrahydro-pyridine-3-carboxylic acid (3,5-bis-trifluoromethylbenzyl)cyclopropylamide;
4-{4-[3-(2,3,6-trifluorophenoxy)propyl]phenyl}-1,2,3,6-tetrahydropyridine-3-carboxylic acid (2-chloro-5-trifluoromethylbenzyl)cyclopropylamide;
4-{4-[3-(2,3,6-trifluorophenoxy)propyl]phenyl}-1,2,3,6-tetrahydropyridine-3-carboxylic acid (2-chloro-3,6-difluorobenzyl)cyclopropylamide; and
4-{4-[3-(2,3,6-trifluorophenoxy)propyl]phenyl}-1,2,3,6-tetrahydropyridine-3-carboxylic acid cyclopropyl-(3-methylbenzyl)amide.

7. Pharmaceutical compositions containing at least one compound of any ones of claims 1 to 6 and usual carrier materials and adjuvants.

8. Use of a compound according to any one of claims 1 to 6 for the preparation of a pharmaceutical composition for the treatment or prophylaxis of diseases which are related to the renin-angiotensin system (RAS) including hypertension, congestive heart failure, pulmonary hypertension, cardiac insufficiency, renal insufficiency, renal or myocardial ischemia, atherosclerosis, renal failure, erectile dysfunction, glomerulonephritis, renal colic, glaucoma, diabetic complications, complications after vascular or cardiac surgery, restenosis, complications of treatment with immunosuppresive agents after organ transplantation, and other diseases which are related to the RAS.

## Patentansprüche

1. Verbindungen der allgemeinen Formel **I** 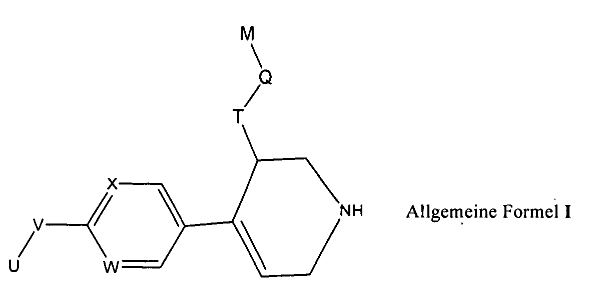 wobei
X und W unabhängig ein Stickstoffatom oder eine CH-Gruppe repräsentieren;
V Folgendes repräsentiert: -(CH₂)ᵣ-; -A-(CH₂)ₛ-; -CH₂-A-(CH₂)ₜ-; -(CH₂)ₛ-A-; -(CH₂)₂-A-(CH₂)ᵤ-; -A-(CH₂)ᵥ-B-; -CH₂-CH₂-CH₂-A-CH₂-; -A-CH₂-CH₂-B-CH₂-; -CH₂-A-CH₂-CH₂-B-; -CH₂-CH₂-CH₂-A-CH₂-CH₂-; -CH₂-CH₂-CH₂-CH₂-A-CH₂-; -A-CH₂-CH₂-B-CH₂-CH₂-; -CH₂-A-CH₂-CH₂-B-CH₂-; -CH₂-A-CH₂-CH₂-CH₂-B- oder -CH₂-CH₂-A-CH₂-CH₂-B-;
A und B unabhängig -O-; -S-; -SO- oder -SO₂- repräsentieren;
U Aryl oder Heteroaryl repräsentiert;
T Folgendes repräsentiert: -CONR¹-; -(CH₂)ₚOCO-; -(CH₂)ₚN(R¹)CO-; -(CH₂)pN(R¹)SO₂-; -COO-; -(CH₂)ₚOCONR¹- oder -(CH₂)ₚN(R¹')CONR¹-;
Q niederes Alkylen oder niederes Alkenylen repräsentiert;
M Wasserstoff; Cycloalkyl; Aryl; Heterocyclyl; Heteroaryl; Aryl-O(CH₂)ᵥR²; Heteroaryl-O(CH₂)ᵥR²; Aryl-O(CH₂)₂O(CH₂)_{w}R²; Heteroaryl-(CH₂)₂O(CH₂)_{w}R²; Aryl-OCH₂CH(R⁵)CH₂R² oder Heteroaryl-OCH₂CH(R⁵)CH₂R² repräsentiert;
R¹ und R¹' unabhängig Wasserstoff; niederes Alkyl; niederes Alkenyl; niederes Alkinyl; Cycloalkyl; Aryl oder Cycloalkyl - niederes Alkyl repräsentieren;
R² Folgendes repräsentiert: -OH, niederes Alkoxy, -OCOR³, -COOR³, -NR³R³', -OCONR³R³', -NCONR³R³', Cyano, -CONR³R³', SO₃H, -SONR³R³', -CO-Morpholin-4-yl, -CO-((4-niederes Alkyl)piperazin-1-yl), -NH(NH)NH₂ oder -NR⁴R⁴', unter der Voraussetzung, dass ein Kohlenstoffatom höchstens an ein Heteroatom angelagert ist, wenn dieses Kohlenstoffatom sp³-hybridisiert ist;
R³ und R³' unabhängig Wasserstoff; niederes Alkyl; niederes Alkenyl; Cycloalkyl oder Cycloalkyl - niederes Alkyl repräsentieren;
R⁴ und R⁴' unabhängig Wasserstoff; niederes Alkyl; Cycloalkyl; Cycloalkyl - niederes Alkyl; Hydroxy - niederes Alkyl; -COOR² oder -CONH₂ repräsentieren;
R⁵ -OH, -OR²; -OCOR² oder -OCOOR² repräsentiert oder R⁵ und R² zusammen mit den Kohlenstoffatomen, an die sie angelagert sind, einen 1,3-Dioxolanring bilden, der in Position 2 durch R³ und R³' substituiert ist, oder R⁵ und R² zusammen mit den Kohlenstoffatomen, an die sie angelagert sind, einen 1,3-Dioxolan-2-on-Ring bilden;
p die ganze Zahl 1, 2, 3 oder 4 ist;
r die ganze Zahl 3, 4, 5 oder 6 ist;
s die ganze Zahl 2, 3, 4 oder 5 ist;
t die ganze Zahl 1, 2, 3 oder 4 ist;
u die ganze Zahl 1, 2 oder 3 ist;
v die ganze Zahl 2, 3 oder 4 ist;
w die ganze Zahl 1 oder 2 ist;
wobei das Präfix "nieder" ein Radikal mit bis zu 7 Kohlenstoffatomen kennzeichnet;
und optisch reine Enantiomere, Gemische von Enantiomeren wie Racemate, Diastereomere, Gemische von Diastereomeren, diastereomere Racemate, Gemische von diastereomeren Racematen und die meso-Form; sowie pharmazeutisch akzeptable Salze, Lösungsmittelkomplexe und morphologische Formen.

2. Verbindungen der allgemeinen Formel **I** nach Anspruch 1, wobei X, W, V und U der Definition in Anspruch 1 entsprechen, wobei
T -CONR¹- repräsentiert;
Q ein Methylen repräsentiert;
M Aryl; Heteroaryl; Aryl-O(CH₂)ᵥR² oder Heteroaryl-O(CH₂)ᵥR² repräsentiert,
und optisch reine Enantiomere, Gemische von Enantiomeren wie Racemate, Diastereomere, Gemische von Diastereomeren, diastereomere Racemate, Gemische von diastereomeren Racematen und die meso-Form; sowie pharmazeutisch akzeptable Salze, Lösungsmittelkomplexe und morphologische Formen.

3. Verbindungen der allgemeinen Formel **I** nach Anspruch 1, wobei X, W, T, Q und M der Definition in Anspruch 1 entsprechen, wobei
V Folgendes repräsentiert:
-CH₂CH₂O-; -CH₂CH₂CH₂O- oder -OCH₂CH₂O-
und U der Definition in Anspruch 1 entspricht,
und optisch reine Enantiomere, Gemische von Enantiomeren wie Racemate, Diastereomere, Gemische von Diastereomeren, diastereomere Racemate, Gemische von diastereomeren Racematen und die meso-Form; sowie pharmazeutisch akzeptable Salze, Lösungsmittelkomplexe und morphologische Formen.

4. Verbindungen der allgemeinen Formel **I** nach Anspruch 1, wobei V, U, T, Q und M der Definition in Anspruch 1 entsprechen, wobei
X und W -CH- repräsentieren;
und optisch reine Enantiomere, Gemische von Enantiomeren wie Racemate, Diastereomere, Gemische von Diastereomeren, diastereomere Racemate, Gemische von diastereomeren Racematen und die meso-Form; sowie pharmazeutisch akzeptable Salze, Lösungsmittelkomplexe und morphologische Formen.

5. Verbindungen der allgemeinen Formel **I** nach Anspruch 1, wobei X, W, V, Q, T und M der Definition in Anspruch 1 entsprechen, wobei
U ein mono-, di- oder trisubstituiertes Phenyl- oder Heteroaryl repräsentiert, wobei die Substituenten unabhängig Halogen, niederes Alkyl, niederes Alkoxy, CF₃ sind,
und optisch reine Enantiomere, Gemische von Enantiomeren wie Racemate, Diastereomere, Gemische von Diastereomeren, diastereomere Racemate, Gemische von diastereomeren Racematen und die meso-Form; sowie pharmazeutisch akzeptable Salze, Lösungsmittelkomplexe und morphologische Formen.

6. Verbindungen nach einem der Ansprüche 1 bis 5, ausgewählt aus der Gruppe bestehend aus
4-{4-[3-(2,3,6-Trifluorphenoxy)propyl]phenyl}-1,2,3,6-tetrahydropyridin-3-carbonsäurecyclopropyl-(2-fluorbenzyl)amid;
4- {4-[3-(2,3,6-Trifluorphenoxy)propyl]phenyl}-1,2,3,6-tetrahydropyridin-3-carbonsäure-(2-chlorbenzyl)ethylamid;
4-{4-[3-(2,3,6-Trifluorphenoxy)propyl]phenyl}-1,2,3,6-tetrahydropyridin-3-carbonsäure-(2-chlorbenzyl)cyclopropylamid;
4- {4-[3-(2,3,6-Trifluorphenoxy)propyl]phenyl}-1,2,3,6-tetrahydropyridin-3-carbonsäurecyclopropyl-(2-fluor-5-methoxybenzyl)amid;
4-{4-[3-(2,3,6-Trifluorphenoxy)propyl]phenyl}-1,2,3,6-tetrahydropyridin-3-carbonsäurecyclopropyl-(3-methoxybenzyl)amid;
4-{4-[3-(2,3,6-Trifluorphenoxy)propyl]phenyl}-1,2,3,6-tetrahydropyridin-3-carbonsäurecyclopropyl-(2-methoxybenzyl)amid;
4-{4-[3-(2,3,6-Trifluorphenoxy)propyl]phenyl}-1,2,3,6-tetrahydro-pyridin-3-carbonsäurecyclopropyl-(5-fluor-2-methoxybenzyl)amid;
4-{4-[3-(2,3,6-Trifluorphenoxy)propyl]phenyl}-1,2,3,6-tetrahydropyridin-3-carbonsäure-(2-chlor-6-fluorbenzyl)cyclopropylamid;
4- {4-[3-(2,3,6-Trifluorphenoxy)propyl]phenyl}-1,2,3,6-tetrahydropyridin-3-carbonsäure-(2-brombenzyl)cyclopropylamid;
4-{4-[3-(2,3,6-Trifluorphenoxy)propyl]phenyl}-1,2,3,6-tetrahydropyridin-3-carbonsäurecyclopropyl-(2,3-dimethylbenzyl)amid;
4- {4-[3-(2,3,6-Trifluorphenoxy)propyl]-henyl}-1,2,3,6-tetrahydro-pyridin-3-carbonsäure-(3,5-bis-trifluormethylbenzyl)cyclopropylamid;
4-{4-[3-(2,3,6-Trifluorphenoxy)propyl]phenyl}-1,2,3,6-tetrahydropyridin-3-carbonsäure-(2-chlor-5-trifluormethylbenzyl)cyclopropylamid;
4-{4-[3-(2,3,6-Trifluorphenoxy)propyl]phenyl}-1,2,3,6-tetrahydropyridin-3-carbonsäure-(2-chlor-3,6-difluorbenzyl)cyclopropylamid und
4-{4-[3-(2,3,6-Trifluorphenoxy)propyl]phenyl}-1,2,3,6-tetrahydropyridin-3-carbonsäurecyclopropyl-(3-methylbenzyl)amid.

7. Pharmazeutische Zusammensetzungen, die wenigstens eine Verbindung nach einem der Ansprüche 1 bis 6 und gewöhnliche Trägermaterialien und Hilfsmittel enthalten.

8. Verwendung einer Verbindung nach einem der Ansprüche 1 bis 6 zur Herstellung einer pharmazeutischen Zusammensetzung zur Behandlung oder Prophylaxe von Krankheiten, die mit dem Renin-Angiotensin-System (RAS) zusammenhängen, wie Hypertonie, kongestive Herzinsuffizienz, pulmonale Hypertonie, Herzinsuffizienz, Niereninsuffizienz, Nieren- oder Myokardischämie, Atherosklerose, Nierenversagen, erektile Fehlfunktion, Glomerulonephritis, Nierenkolik, Glaukom, diabetische Komplikationen, Komplikationen nach einer Gefäß- oder Herzoperation, Restenose, Komplikationen bei einer Behandlung mit immunsuppressiven Mitteln nach einer Organtransplantation und andere Krankheiten, die mit dem RAS zusammenhängen.

## Revendications

1. Composés de la formule générale **I** 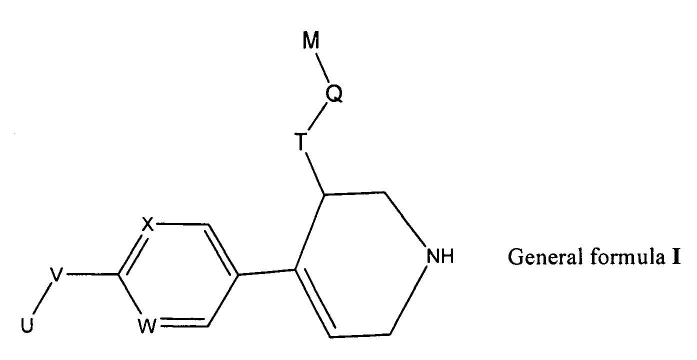 General formula I = Formule générale I
où
X et W représentent indépendamment un atome d'azote ou un groupement CH-;
V représente -(CH₂)ᵣ-; -A-(CH₂)ₛ-; -CH₂-A-(CH₂)ₜ-; -(CH₂)ₛ-A-; -(CH₂)₂-A-(CH₂)ᵤ-; -A-(CH₂)ᵥ-B-; -CH₂-CH₂-CH₂-A-CH₂-; -A-CH₂-CH₂-B-CH₂-; -CH₂-A-CH₂-CH₂-B-; -CH₂-CH₂-CH₂-A-CH₂-CH₂-; -CH₂-CH₂-CH₂-CH₂-A-CH₂-; -A-CH₂-CH₂-B-CH₂-CH₂-; -CH₂-A-CH₂-CH₂-B-CH₂-; -CH₂-A-CH₂-CH₂-CH₂-B-; ou -CH₂-CH₂-A-CH₂-CH₂-B-;
A et B représentent indépendamment -O-; -S-; -SO-; ou -SO₂-;
U représente un groupement aryle; ou un groupement hétéroaryle;
T représente -CONR¹-; -(CH₂)ₚOCO-; -(CH₂)ₚN(R¹)CO-; -(CH₂)ₚN(R¹)SO₂-; -COO-; -(CH₂)ₚOCONR¹-; ou -(CH₂)ₚN(R¹')CONR¹-;
Q représente un groupement alcylène inférieur; ou un groupement alcénylène inférieur;
M représente l'hydrogène; un radical cycloalkyle; un radical aryle; un radical hétérocyclyle; hétéroaryle; aryl-O(CH₂)ᵥR²; hétéroaryl-O(CH₂)ᵥR²; aryl-O(CH₂)O(CH₂)_{w}R²; hétéroaryl-(CH₂)₂O(CH₂)_{w}R²; aryl-OCH₂CH(R⁵)CH₂R²; ou hétéroaryl-OCH₂CH(R⁵)CH₂R²;
R¹ et R¹' représentent indépendamment l'hydrogène; un radical alkyle inférieur; un radical alcényle inférieur; un radical alcynyle inférieur; un radical cycloalkyle; un radical aryle; ou un radical cycloalkyl-alkyle inférieur;
R² représente un radical -OH, alkoxy inférieur, -OCOR³, -COOR³, -NR³R³', -OCONR³R³, -NCONR³R³', cyano, -CONR³R³', SO₃H, -SONR³R^{3'}, -CO-morpholin-4-yl, -CO-((4-alkyle inférieur)pipérazin-1-yl), -NH(NH)NH₂, ou -NR⁴R⁴', sous réserve qu'un atome de carbone soit attaché au plus à un hétéro-atome au cas où cet atome de carbone présente une hybridation sp³;
R³ et R^{3'} représentent indépendamment l'hydrogène; un radical alkyle inférieur; un radical alcényle inférieur; un radical cycloalkyle; ou un radical cycloalkyl-alkyle inférieur;
R⁴ et R^{4'} représentent indépendamment l'hydrogène; un radical alkyle inférieur; un radical cycloalkyle; un radical cycloalkyl-alkyle inférieur; un radical hydroxy-alkyle inférieur; -COOR²; ou -CONH₂;
R⁵ représente un radical -OH, -OR²; -OCOR²; ou -OCOOR²; ou R⁵ et R² forment ensemble avec les atomes de carbone auxquels ils sont attachés un cycle 1,3-dioxolanne qui est substitué en position 2 par R³ et R³'; ou R⁵ et R² forment ensemble avec les atomes de carbone auxquels ils sont attachés un cycle 1,3-dioxolan-2-one;
p est l'entier 1, 2, 3 ou 4;
r est l'entier 3, 4, 5 ou 6;
s est l'entier 2, 3, 4 ou 5;
t est l'entier 1, 2, 3 ou 4;
u est l'entier 1, 2, ou 3;
v est l'entier 2, 3, ou 4;
w est l'entier 1 ou 2;
et où le suffixe "inférieur" dénote un radical ayant jusqu'à 7 atomes de carbone;
et des énantiomères optiquement purs, des mélanges d'énantiomères, comme les racémates, les diastéréomères, les mélanges de diastéréoisomères, les racémates diastéréomères, les mélanges de racémates diastéréomères, et la forme méso, ainsi que des sels acceptables du point de vue pharmaceutique, des complexes à solvants et des formes morphologiques.

2. Composés de la formule générale **I** selon la revendication 1 dans lesquels X, W, V, et U sont définis comme dans la revendication 1, où
T représente -CONR¹-;
Q représente un groupement méthylène;
M représente un radical aryle; hétéroaryle; aryl-O(CH₂)ᵥR²; ou hétéroaryl-O(CH₂)ᵥR²;
et des énantiomères optiquement purs, des mélanges d'énantiomères, comme les racémates, les diastéréomères, les mélanges de diastéréoisomères, les racémates diastéréomères, les mélanges de racémates diastéréomères, et la forme méso, ainsi que des sels acceptables du point de vue pharmaceutique, des complexes à solvants et des formes morphologiques.

3. Composés de la formule générale **I** selon la revendication 1 dans lesquels X, W, T, Q et M sont définis comme dans la revendication 1, où
V représente
-CH₂CH₂O-; -CH₂CH₂CH₂O-; ou -OCH₂CH₂O-;
et U est comme défini dans la revendication 1
et des énantiomères optiquement purs, des mélanges d'énantiomères, comme les racémates, les diastéréomères, les mélanges de diastéréoisomères, les racémates diastéréomères, les mélanges de racémates diastéréomères, et la forme méso, ainsi que des sels acceptables du point de vue pharmaceutique, des complexes à solvants et des formes morphologiques.

4. Composés de la formule générale **I** selon la revendication 1 dans lesquels V. U, T, Q et M sont comme définis dans la revendication 1, où
X et W représentent -CH-.
et des énantiomères optiquement purs, des mélanges d'énantiomères, comme les racémates, les diastéréomères, les mélanges de diastéréoisomères, les racémates diastéréomères, les mélanges de racémates diastéréomères, et la forme méso, ainsi que des sels acceptables du point de vue pharmaceutique, des complexes à solvants et des formes morphologiques.

5. Composés de la formule générale **I** selon la revendication 1 dans lesquels X, W, V, Q, T et M sont comme définis dans la revendication 1, où
U représente un groupement phényle ou un groupement hétéroaryle mono-, di- ou tri-substitué, où les substituants sont indépendamment un halogène, un alkyle inférieur, un alkoxy inférieur, CF₃;
et des énantiomères optiquement purs, des mélanges d'énantiomères, comme les racémates, les diastéréomères, les mélanges de diastéréoisomères, les racémates diastéréomères, les mélanges de racémates diastéréomères, et la forme méso, ainsi que des sels acceptables du point de vue pharmaceutique, des complexes à solvants et des formes morphologiques.

6. Les composés selon l'une quelconque des revendications 1 à 5, sélectionnés parmi le groupe constitué
du cyclopropyl-(2-fluorobenzyl)amide de l'acide 4-{4-[3-(2,3,6-trifluorophénoxy)propyl]phényl}-1,2,3,6-tétrahydropyridine-3-carboxylique;
du (2-chlorobenzyl)ethylamide de l'acide 4-{4-[3-(2,3,6-trifluorophénoxy)propyl]phényl} -1,2,3,6-tétrahydropyridine-3-carboxylique;
du (2-chlorobenzyl)cyclopropylamide de l'acide 4-{4-[3-(2,3,6-trifluorophénoxy)propyl]phényl}-1,2,3,6-tétrahydropyridine-3-carboxylique;
du cyclopropyl-(2-fluoro-5-méthoxybenzyl)amide de l'acide 4-{4-[3-(2,3,6-trifluorophénoxy)propyl]phényl}-1,2,3,6-tétrahydropyridine-3-carboxylique;
du cyclopropyl-(3-méthoxybenzyl)amide de l'acide 4-{4-[3-(2,3,6-trifluorophénoxy)propyl]phényl}-1,2,3,6-tétrahydropyridine-3-carboxylique ;
du cyclopropyl-(2-méthoxybenzyl)amide de l'acide 4-{4-[2-(2,3,6-trifluorophénoxy)propyl]phényl}-1,2,3,6-tétrahydropyridine-3-carboxylique ;
du cyclopropyl-(5-fluoro-2-méthoxybenzyl)amide de l'acide 4-{4-[3-(2,3,6-trifluorophénoxy)propyl]phényl}-1,2,3,6-tétrahydro-pyridine-3-carboxylique;
du (2-chloro-6-fluorobenzyl)cyclopropylamide de l'acide 4-{4-[3-(2,3,6-trifluorophénoxy)propyl]phényl}-1,2,3,6-tétrahydropyridine-3-carboxylique;
du (2-bromobenzyl)cyclopropylamide de l'acide 4-{4-[3-(2,3,6-trifluorophénoxy)propyl]phényl} -1,2,3,6-tétrahydropyridine-3-carboxylique;
du cyclopropyl-(2,3-diméthylbenzyl)amide de l'acide 4-{4-[3-(2,3,6-trifluorophénoxy)propyl]phényl }-1,2,3,6-tétrahydropyridine-3-carboxylique;
du (3,5-bis-trifluorométhylbenzyl)cyclopropylamide de l'acide 4-{4-[3-(2,3,6-trifluorophénoxy)propyl]-phényl}-1,2,3,6-tétrahydro-pyridine-3-carboxylique;
du (2-chloro-5-trifluorométhylbenzyl)cyclopropylamide de l'acide 4-{4-[3-(2,3,6-trifluorophénoxy)propyl]phényl}-1,2,3,6-tétrahydropyridine-3-carboxylique;
du (2-chloro-3,6-difluorobenzyl)cyclopropylamide de l'acide 4-{4-[3-(2,3,6-trifluorophénoxy)propyl]phényl}-1,2,3,6-tétrahydropyridine-3-carboxylique; et
du cyclopropyl-(3-méthylbenzyl)amide de l'acide 4-{4-[3-(2,3,6-trifluorophénoxy)propyl]phényl}-1,2,3,6-tétrahydropyridine-3-carboxylique.

7. Compositions pharmaceutiques contenant au moins un composé selon l'une quelconque des revendications 1 à 6 et des excipients et adjuvants usuels.

8. Utilisation d'un composé selon l'une quelconque des revendications 1 à 6 en vue de la préparation d'une composition pharmaceutique pour le traitement ou la prophylaxie de maladies qui sont associées au système rénine-angiotensine (RAS), y compris l'hypertension, l'insuffisance cardiaque congestive, l'hypertension pulmonaire, l'insuffisance cardiaque, l'insuffisance rénale, l'ischémie rénale ou myocardique, l'athérosclérose, l'insuffisance rénale, la dysfonction érectile, la glomérulonéphrite, la colique rénale, le glaucome, les complications diabétiques, les complications après chirurgie vasculaire ou cardiaque, la resténose, des complications du traitement à l'aide d'agents immunosuppressifs après transplantation d'organes, et d'autres maladies qui sont associées au RAS.
